(19) Europäisches Patentamt / European Patent Office / Office européen des brevets

(11) **EP 3 643 274 A1**

(12) **EUROPEAN PATENT APPLICATION**
published in accordance with Art. 153(4) EPC

(43) Date of publication:
**29.04.2020 Bulletin 2020/18**

(21) Application number: **18873052.7**

(22) Date of filing: **18.04.2018**

(51) Int Cl.:
***A61F 2/90*** (2013.01)

(86) International application number:
**PCT/JP2018/016040**

(87) International publication number:
**WO 2019/087433 (09.05.2019 Gazette 2019/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.10.2017 JP 2017210753**

(71) Applicant: **Japan Lifeline Co., Ltd.**
**Tokyo 140-0002 (JP)**

(72) Inventors:
• **KUWAHARA Kunio**
**Tokyo 140-0002 (JP)**
• **NAKANO Eiichi**
**Tokyo 140-0002 (JP)**

(74) Representative: **D Young & Co LLP**
**120 Holborn**
**London EC1N 2DY (GB)**

(54) **STENT AND MEDICAL DEVICE**

(57) Provided is a stent, for example, that enables an improvement in characteristics. A stent 11 includes: a first mesh structure (mesh structure 111) that includes one or more first wire members (wire members W1) and has a tubular structure extending over an entire length in an axial direction Z of the stent 11; and a second mesh structure (mesh structure 112) that is disposed in at least a portion of the stent 11 and includes one or more second wire members (wire members W2) intersecting the first wire members of the first mesh structure. The first wire members are coupled to each other to form a coupling part (coupling parts C11 and C12) in the first mesh structure, and the second wire members are not coupled to each other in the second mesh structure. Moreover, the first wire member has a wire diameter r1 that is smaller than a wire diameter r2 of the second wire member (r1 < r2).

[ FIG. 2 ]

(WIRE DIAMETER r1 OF W1 < WIRE DIAMETER r2 OF W2)

**Description**

Technical Field

The invention relates to a stent to be applied, for example, to a tubular organ in a body, such as a digestive tract or a blood vessel, and also relates to a medical device, such as a covered stent or a stent graft, that includes such a stent.

Background Art

A stent to be applied to (placed in) a digestive tract (a digestive tract stent) is used to push open a lumen of the digestive tract that is narrowed by a tumor. Moreover, stents are also used for other tubular organs in the body, such as the blood vessel, in addition to the digestive tract. Such a stent to be applied to a tubular organ in the body generally has a mesh structure including one or more wire members (e.g., see PTL 1).

Citation List

Patent Literature

PTL 1: Japanese Unexamined Patent Application Publication (Published Japanese Translation of PCT Application) No. JP2009-501049

Summary of Invention

Incidentally, it is generally demanded that such a stent be improved in various characteristics. Therefore, it is desirable to provide a stent that enables an improvement in characteristics and a medical device that includes such a stent.

A stent according to one embodiment of the invention includes: a first mesh structure that includes one or more first wire members and has a tubular structure extending over an entire length in an axial direction of the stent; and a second mesh structure that is disposed in at least a portion of the stent and includes one or more second wire members intersecting the first wire members of the first mesh structure. The first wire members are coupled to each other to form a coupling part in the first mesh structure, and the second wire members are not coupled to each other in the second mesh structure. Moreover, the first wire member has a wire diameter that is smaller than a wire diameter of the second wire member.

It is to be noted that "coupling" as used herein refers to a state in which bends in wire members are hooked to (engaged with) each other or a bend in a wire member and an intersection of wire members are hooked to each other.

A medical device according to one embodiment of the invention includes: a tubular member; and at least one stent according to one embodiment of the invention described above, the stent being disposed in at least a portion of the tubular member.

In the stent and the medical device according to one embodiment of the invention, the wire diameter of the first wire member constituting the first mesh structure (the tubular structure) is smaller than the wire diameter of the second wire member constituting the second mesh structure (the wire diameter of the first wire member < the wire diameter of the second wire member). This makes it easier to well-balance various characteristics of the stent that vary depending on magnitudes of the wire diameters.

In the stent and the medical device according to one embodiment of the invention, in both ends along the axial direction of the stent, the first wire member constituting the first mesh structure (the tubular structure) may be disposed, whereas the second wire member constituting the second mesh structure may not be disposed. In such a case, expansion force of the stent in the both ends is weakened, which makes it less likely for a digestive tract, a blood vessel, etc. to be damaged in these both ends. This improves reliability of the stent.

Moreover, the second mesh structure may include a unit structure and a gap region that are provided alternately along the axial direction of the stent, and in this gap region, the first wire member constituting the first mesh structure (the tubular structure) may be disposed, whereas the second wire member constituting the second mesh structure may not be disposed. In such a case, moderate digestive tract conformability to a tubular organ is maintained while the expansion force of the entire stent is kept. This further improves the characteristics of the stent.

According to the stent and the medical device according to one embodiment of the invention, the wire diameter of the first wire member is made smaller than the wire diameter of the second wire member, which makes it possible to well-balance the various characteristics of the stent that vary depending on the magnitudes of the wire diameters. This makes it possible to improve the characteristics of the stent.

Brief Description of Drawings

[FIG. 1] FIG. 1 is a schematic perspective view of an outline configuration example of a stent according to one embodiment of the invention.
[FIG. 2] FIG. 2 is a schematic development view of a detailed configuration example of a main part of the stent illustrated in FIG. 1.
[FIG. 3] FIG. 3 is a diagram illustrating an example of a correspondence between a wire diameter of a wire member and various characteristics in a typical stent.
[FIG. 4] FIG. 4 is a schematic development view of a configuration example of a main part of a stent according to a modification example.
[FIG. 5] FIG. 5 is a schematic perspective view of an outline configuration example of a medical device according to an application example.

Description of Embodiments

Hereinafter, embodiments of the invention will be described in detail with reference to the drawings. It is to be noted that the description will be given in the following order.

1. Embodiment (an example of a stent including two types of wire members with different wire diameters)
2. Modification Example (an example of a stent including another mesh structure different from that in the embodiment)
3. Application Example (an example in a case where the stents of the embodiment and the modification example are applied to a medical device)
4. Other Modification Examples

<1. Embodiment>

[Outline Configuration]

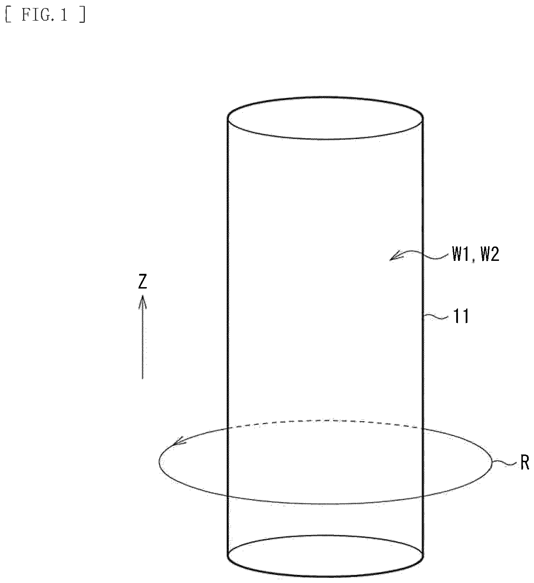

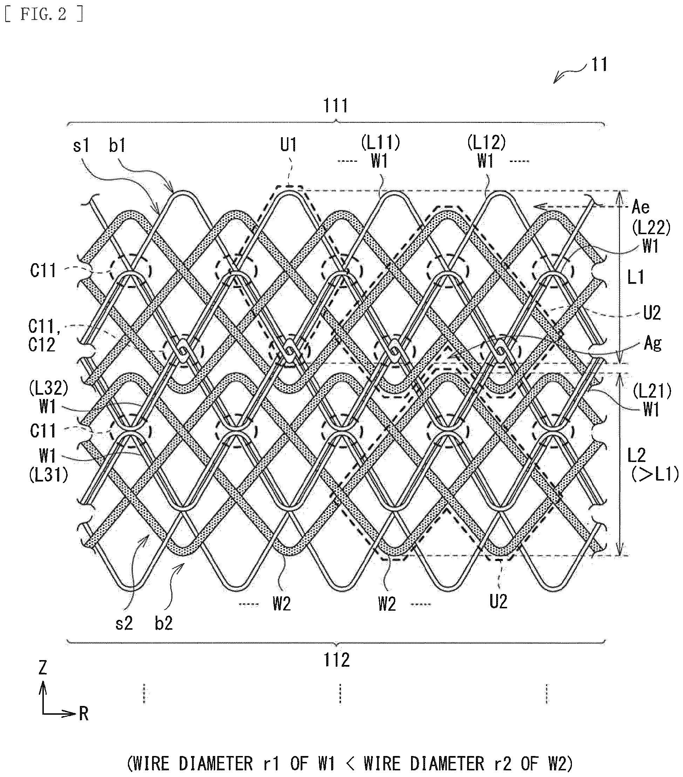

FIG. 1 is a schematic perspective view of an outline configuration example of a stent (a stent 11) according to one embodiment of the invention. The stent 11 is an instrument to be applied, for example, to a digestive tract serving as a tubular organ in a body, and is used to push open a lumen of the digestive tract that is narrowed by a tumor, as will be described later. Specifically, the stent 11 is to be placed in a part to be treated (e.g., in the digestive tract such as a large intestine). It is to be noted that the stent 11 corresponds to a specific example of a "stent" in the invention.
The stent 11 has a tubular (cylindrical) structure that extends along its axial direction (Z axis direction), as illustrated in FIG 1. The stent 11 has a length along the axial direction Z of, for example, about 3 cm to about 20 cm. On the other hand, the stent 11 has, when expanded, an outer diameter (a length along a circumferential direction R) of, for example, about 10 mm to about 50 mm.
As illustrated in FIG. 1, the stent 11 includes two types of wire members W1 and W2 (wires), and has a tubular structure as described above. Specifically, in the present embodiment, the tubular structure includes a mesh structure, and such a tubular mesh structure is formed by weaving each of the wire members W1 and W2 in a predetermined pattern (a mesh pattern to be described later). It is to be noted that the mesh structure (each of weaving patterns of the wire members W1 and W2) etc. in the stent 11 will be described in detail later (FIG. 2).
In this example, a metal wire member may be preferable as a material of such wire members W1 and W2. In particular, a shape-memory alloy may be preferably employed. The shape-memory alloy is provided with a shape-memory effect, superelasticity, etc. by a thermal process. However, stainless-steel, tantalum (Ta), titanium (Ti), platinum (Pt), gold (Au), tungsten (W), etc. may be used as the material of the wire member W1, depending on the application. As the above-described shape-memory alloy, for example, an alloy of nickel (Ni) and Ti, an alloy of copper (Cu), zinc (Zn), and X (X is aluminum (Al), iron (Fe), etc.), an alloy of Ni, Ti, and X (X is Fe, Cu, vanadium (V), cobalt (Co), etc.), etc. may be preferably used. It is to be noted that, for example, a synthetic resin, etc. may be used as such wire members W1 and W2. Moreover, a metal wire member having a surface covered with Au, Pt, etc. by a method such as plating may be used as the wire members W1 and W2. Alternatively, a complex wire member in which a core material is covered with an alloy may be used as the wire members W1 and W2. The core material includes a material that does not transmit radiation, such as Au or Pt.
It is to be noted that the wire member W1 corresponds to a specific example of a "first wire member" in the invention. Moreover, the wire member W2 corresponds to a specific example of a "second wire member" in the invention.

[Detailed Configuration]

Next, with reference to FIG. 2, description will be given on a detailed configuration example of the stent 11 illustrated in FIG. 1 (a configuration example of the above-described mesh structure and the like). FIG. 2 is a schematic development view of a detailed configuration example of a main part (the vicinity of an end region Ae) of the stent 11, and illustrates the detailed configuration example along each of the axial direction Z and the circumferential direction R illustrated in FIG. 1.

It is to be noted that, for convenience, the mesh structure including the wire member W1 (a mesh structure 111 to be described later) is not hatched in FIG. 2. In contrast, the mesh structure including the wire member W2 (a mesh structure 112 to be described later) is hatched in FIG. 2.

Here, firstly, the stent 11 of the present embodiment includes the two types of mesh structures 111 and 112, as illustrated in FIG. 2. That is, the stent 11 includes the mesh structure 111 including the wire member W1 and the mesh structure 112 including the wire member W2. Of these, the mesh structure 111 constitutes the above-described tubular structure and extends over the entire length in the axial direction Z of the stent 11. In other words, this mesh structure 111 has no gap region along the axial direction Z, unlike the mesh structure 112. In contrast, the mesh structure 112 is disposed in at least a portion (a portion in the example of FIG. 2) of the stent 11, and has a gap region Ag along the axial direction Z as illustrated in FIG. 2. This will be described in detail later.

Moreover, in the stent 11, as illustrated in FIG 2, the wire members W1 and W2 respectively constituting such two types of mesh structures 111 and 112 have wire diameters r1 and r2 that are different from each other (the wire diameter r1 of the wire member W1 ≠ the wire diameter r2 of the wire member W2). In other words, the stent 11 includes the two types of wire members W1 and W2 with the different wire diameters r1 and r2. Moreover, particularly in the stent 11 of the present embodiment, as illustrated in FIG. 2, the wire diameter r1 of the wire member W1 constituting the mesh structure 111 (the tubular structure) is smaller (thinner) than the wire diameter r2 of the wire member W2 constituting the mesh structure 112 (the wire diameter r1 < the wire diameter r2). To put it another way, the wire diameter r2 of the wire member W2 is larger (thicker) than the wire diameter r1 of the wire member W1. It is to be noted that an example of such a wire diameter r1 is about 0.18 mm, and an example of such a wire diameter r2 is about 0.20 mm.

Hereinafter, configuration examples of such mesh structures 111 and 112 will be described in detail, in the order of the mesh structures 111 and 112.

(Basic Configuration of Mesh Structure 111)

Firstly, the stent 11 of the present embodiment includes the mesh structure 111, which is a structure extending along each of the axial direction Z and the circumferential direction R, as illustrated in FIG. 2. The mesh structure 111 includes one or more wire members (one wire member W1 in this example) that include straight parts s1 and bends b1 and form a wavy shape (zigzag shape).

It is to be noted that such a mesh structure 111 corresponds to a specific example of a "first mesh structure" (main stent) in the invention.

Here, a plurality of rows of mesh patterns is formed in this mesh structure 111. The rows are adjacent to each other along the axial direction Z. Moreover, in the mesh pattern of each row (one row), the wire members W1 are arranged so as to intersect each other (so as to form intersections) at their straight parts s1, and the wire members W1 make loops in the circumferential direction R to allow the intersections to be located side by side (scattered) along the circumferential direction R. In addition, the wire members W1 constituting such a mesh pattern of one row each repeatedly progress (move) while being sequentially shifted in the axial direction Z, to form the plurality of rows of mesh patterns as described above.

Specifically, such a mesh pattern of each row (one row) is formed by the wire member W1 making two laps of loops along the circumferential direction R while forming the above-described wavy shape. More specifically, the mesh pattern of each row includes a first loop as the loop of the first lap (e.g., loops L11, L21, L31, etc. to be described later) and a second loop as the loop of the second lap (e.g., loops L12, L22, L32, etc. to be described later). In the mesh pattern of each row, the first loop and the second loop have wavy shapes whose phases are shifted by half a pitch (1/2 pitches) from each other along the circumferential direction R. Thus, in the mesh pattern of each row, the above-described intersections are located side by side along the circumferential direction R. It is to be noted that, in such intersections, the first loop and the second loop intersect so as to go up and down alternately.

Moreover, the mesh patterns of rows that are adjacent to each other along the axial direction Z have wavy shapes whose phases are shifted by 1/4 pitches from each other along the circumferential direction R. Specifically, if the end along the axial direction Z of the mesh structure 111 (the end region Ae) is regarded as a starting point, the mesh pattern of the n-th row (n: natural number) and the mesh pattern of the (n+1)-th row have wavy shapes whose phases are shifted by 1/4 pitches from each other along the circumferential direction R. Similarly, the mesh pattern of the (n+1)-th row and the mesh pattern of the (n+2)-th row have wavy shapes whose phases are shifted by 1/4 pitches from each other along the circumferential direction R. Consequently, the mesh pattern of the n-th row and the mesh pattern of the (n+2)-th row have wavy shapes whose phases are shifted by 1/2 pitches from each other along the circumferential direction R. It is to be noted that, between the mesh pattern of the n-th row and the mesh pattern of the (n+2)-th row, positions of the bends b1 in the first loops match each other along the circumferential direction R, and positions of the bends b1 in the second loops match each other along the circumferential direction R.

Referring now to FIG. 2, more detailed description will be given on the plurality of rows of mesh patterns in such a mesh structure 111, taking as an example the mesh patterns of three rows (first to third rows) from starting points. The starting points are the both ends along the axial direction Z of the mesh structure 111 (both end regions Ae). It is to be noted that the mesh patterns of the fourth and subsequent rows are similar to the mesh patterns of the first to third rows.

It is to be noted that, in the following, it is assumed that the mesh patterns of the first rows from the above-described both ends each include the loop L11 as the first loop and the loop L12 as the second loop. Similarly, it is assumed that the mesh patterns of the second rows from the both ends each include the loop L21 as the first loop and the loop L22 as the second loop. Moreover, it is assumed that the mesh patterns of the third rows from the both ends each include the loop L31 as the first loop and the loop L32 as the second loop.

Firstly, as illustrated in FIG. 2, in the mesh pattern of the first row, the loop L11 and the loop L12 have wavy shapes whose phases are shifted by 1/2 pitches from each other along the circumferential direction R. Similarly, in the mesh pattern of the second row, the loop L21 and the loop L22 have wavy shapes whose phases are shifted by 1/2 pitches from each other along the circumferential direction R. In the mesh pattern of the third row, the loop L31 and the loop L32 have wavy shapes whose phases are shifted by 1/2 pitches from each other along the circumferential direction R. Thus, in each of the mesh patterns of the first to third rows, the above-described intersections are located side by side along the circumferential direction R. It is to be noted that, in such intersections, the loops L11 and L12 intersect so as to go up and down alternately, the loops L21 and L22 intersect so as to go up and down alternately, and the loops L31 and L32 intersect so as to go up and down alternately, as illustrated in FIG. 2.

Moreover, as illustrated in FIG. 2, the mesh pattern of the first row and the mesh pattern of the second row have wavy shapes whose phases are shifted by 1/4 pitches from each other along the circumferential direction R. Similarly, the mesh pattern of the second row and the mesh pattern of the third row have wavy shapes whose phases are shifted by 1/4 pitches from each other along the circumferential direction R. Consequently, the mesh pattern of the first row and the mesh pattern of the third row have wavy shapes whose phases are shifted by 1/2 pitches from each other along the circumferential direction R. It is to be noted that, between the mesh pattern of the first row and the mesh pattern of the third row, positions of the bends b1 in the loops L11 and L31 match each other along the circumferential direction R, and positions of the bends b1 in the loops L12 and L32 match each other along the circumferential direction R. In other words, as illustrated in FIG. 2, the bend b1 in the loop L11 and the bend b1 in the loop L31 are arranged to be adjacent to each other along the axial direction Z. Similarly, the bend b1 in the loop L12 and the bend b1 in the loop L32 are arranged to be adjacent to each other along the axial direction Z.

(Coupling Part C11)

Moreover, such a mesh structure 111 has the following coupling parts (hooking parts) C11 formed in the entirety of its formation region along the axial direction Z. That is, in this mesh structure 111, between the mesh patterns of rows that are adjacent to each other along the axial direction Z, the coupling parts C11 are formed by the respective above-described intersections and the respective bends b1 being coupled to each other. It is to be noted that, in each of such coupling parts C11, the bend b1 is located closer to the outside (an outer circumference) than the intersection is, as illustrated in FIG. 2.

Specifically, the coupling part C11 is formed by the intersection in the mesh pattern of the n-th row and the bend b1 in the mesh pattern of the (n+1)-th row being coupled to each other to allow the bend b1 to be located closer to the outer circumference than the intersection is. Similarly, the coupling part C11 is formed by the intersection in the mesh pattern of the (n+1)-th row and the bend b1 in the mesh pattern of the (n+2)-th row being coupled to each other to allow the bend b1 to be located closer to the outer circumference than the intersection is. Moreover, the coupling part C11 is also formed by the intersection in the mesh pattern of the (n+1)-th row and the bend b1 in the mesh pattern of the n-th row being coupled to each other to allow the bend b1 to be located closer to the outer circumference than the intersection is. Similarly, the coupling part C11 is also formed by the intersection in the mesh pattern of the (n+2)-th row and the bend b1 in the mesh pattern of the (n+1)-th row being coupled to each other to allow the bend b1 to be located closer to the outer circumference than the intersection is. By being formed in the entire region of the mesh structure 111, such coupling parts C11 avoid the possibility that the wire member W1 may protrude to an inner circumference side (lumen side) in a state in which the stent 11 is bent.

Referring now to FIG. 2, more detailed description will be given on the coupling parts C11 in such a mesh structure 111, taking as an example the mesh patterns of the three rows (first to third rows) from the starting points. The starting points are the both ends along the axial direction Z of the mesh structure 111 (the both end regions Ae).

Firstly, as illustrated in FIG. 2, the coupling part C11 is formed by the intersection in the mesh pattern of the first row and the bend b1 in the mesh pattern of the second row being coupled to (engaged with) each other. Similarly, the coupling part C11 is formed by the intersection in the mesh pattern of the second row and the bend b1 in the mesh pattern of the third row being coupled to each other. Moreover, the coupling part C11 is also formed by the intersection in the mesh pattern of the second row and the bend b1 in the mesh pattern of the first row being coupled to each other. Similarly, the coupling part C11 is also formed by the intersection in the mesh pattern of the third row and the bend b1 in the mesh pattern of the second row being coupled to each other.

It is to be noted that such a coupling part C11 corresponds to a specific example of a "coupling part" (first coupling part) in the invention.

(Coupling Part C12)

Here, in the mesh structure 111 of the present embodiment, the following coupling parts C12 (coupling between the bends b1) are further formed in a portion of the formation region, in addition to the above-described coupling parts C11 (coupling between the intersections and the bends b1).

Specifically, as illustrated in FIG. 2, in this mesh structure 111, the coupling parts C12 are formed as follows from the starting point. The starting point is at least one end (in this example, the both end regions Ae) of the both ends along the axial direction Z of the mesh structure 111 (the both end regions Ae). That is, the coupling parts C12 are formed by the respective bends b1 in the mesh pattern of the first row and the respective bends b1 in the mesh pattern of a predetermined odd-numbered row out of the third and subsequent odd-numbered rows being coupled to each other. Moreover, it is desirable that the mesh pattern of the predetermined odd-numbered row be, for example, the mesh pattern of the third or fifth row. In other words, it is desirable that the coupling parts C12 be formed by the respective bends b1 in the mesh pattern of the first row and the respective bends b1 in the mesh pattern of the third or fifth row being coupled to each other.

In the example of FIG. 2, more specifically, the above-described mesh pattern of the predetermined odd-numbered row is the mesh pattern of the third row. In other words, in the example of FIG. 2, the coupling part C12 is formed by the bend b1 in the mesh pattern of the first row (the loop L11 or the loop L12) and the bend b1 in the mesh pattern of the third row (the loop L31 or the loop L32) being coupled to each other in a formation region of the coupling part C11.

It is to be noted that such a coupling part C12 also corresponds to a specific example of a "coupling part" (second coupling part) in the invention.

In contrast, as illustrated in FIG. 2, of the formation region along the axial direction Z of the mesh structure 111, in a region (intermediate region) excluding the vicinity of the above-described at least one end (the end region Ae), only the coupling parts C11 (coupling between the intersections and the bends b1) are formed, and the coupling parts C12 (coupling between the bends b1) are not formed. In this manner, in the mesh structure 111, the coupling parts C12 (the coupling parts between the bends b1 in the mesh patterns of the first and third rows) are selectively formed in the vicinity of the at least one end (in this example, the both end regions Ae).

Moreover, in the present embodiment, in the formation region of the coupling part C11 in the vicinity of the at least one end (in this example, the both end regions Ae), the coupling part C12 is formed at the following position. That is, as illustrated in FIG. 2, the coupling part C12 (e.g., the coupling part between the bends b1 in the mesh patterns of the first and third rows) is formed closer to the outer circumference than the intersection in the mesh pattern (e.g., the intersection of the loops L21 and L22 of the mesh pattern of the second row) is.

(Unit Structure U1)

Furthermore, such a mesh structure 111 includes a plurality of unit structures U1 (first unit structures) arranged side by side along each of the axial direction Z and the circumferential direction R, as illustrated in FIG. 2. In the example of FIG. 2, each unit structure U1 includes a region that is surrounded by a plurality of loops (e.g., any of the loops L11, L12, L21, L22, L31, and L32) in the wire member W1. Specifically, each unit structure U1 has a substantially diamond shape whose vertices are the bends b1 and the above-described intersections. Moreover, in this example, the unit structure U1 has a length along the axial direction Z (an axial direction length L1: for example, about 8 mm to about 24 mm) that substantially matches a wave height of the mesh pattern of each row (e.g., the loops L11, L12, L21, L22, L31, and L32).

Next, the configuration example of the mesh structure 112 will be described.

(Basic Configuration of Mesh Structure 112)

The mesh structure 112 includes one or more wire members W2 (one wire member W2 in this example) that intersect the wire member W1. Specifically, as illustrated in FIG. 2, the wire member W2 includes straight parts s2 and bends b2 and forms a wavy shape (zigzag shape). The mesh structure 112 is formed by the wire member W2 intersecting at its straight parts s2. Therefore, also in the mesh structure 112, intersections (wire member intersections) are formed as in the mesh structure 111. Each of the intersections is a portion where the straight parts s2 of the wire member W2 intersect each other. Moreover, such a mesh structure 112 is woven into the mesh structure 111, by the straight part s2 of the wire member W2 and the straight part s1 of the wire member W1 intersecting each other. The wire members W2 make loops in the circumferential direction R to allow such intersections of the wire members W2 to be located side by side (scattered) along the circumferential direction R. In addition, the wire members W2 constituting the mesh pattern of one row each repeatedly progress (move) while being sequentially shifted in the axial direction Z, to form the plurality of rows of mesh patterns.

Referring now to FIG. 2, more detailed description will be given on the plurality of rows (respective rows) of mesh patterns in such a mesh structure 112, taking as an example the mesh patterns of two rows (first and second rows) from starting points. The starting points are the both ends along the axial direction Z of the mesh structure 112 (both end regions Ae).

The mesh pattern of each row in the mesh structure 112 includes three types of loops (a first loop, a second loop, and a third loop) that are made along the circumferential direction R to be contiguous with each other, as illustrated in FIG. 2. In the mesh pattern of each row, the first loop and the second loop have wavy shapes whose phases are shifted by 1/3 pitches from each other along the circumferential direction R. Similarly, in the mesh pattern of each row, the second loop and the third loop have wavy shapes whose phases are shifted by 1/3 pitches from each other along the circumferential direction R. Thus, in the mesh pattern of each row, the above-described intersections of the wire members W2 are located side by side along the circumferential direction R.

Here, as described above, the wire members W1 of the mesh structure 111 are coupled to each other. In other words, the coupling parts C11 (coupling between the intersections and the bends b1) and the coupling parts C12 (coupling between the bends b1) are formed. In contrast, as illustrated in FIG. 2, the wire members W2 of the mesh structure 112 intersect each other, but are not coupled to each other (a coupling part like the coupling parts C11 and C12 is not formed).

It is to be noted that the mesh structure 112 corresponds to a specific example of a "second mesh structure" (sub-stent) in the invention.

(Unit Structure U2)

Moreover, such a mesh structure 112 includes a plurality of unit structures U2 (second unit structures) arranged side by side along each of the axial direction Z and the circumferential direction R, as illustrated in FIG. 2. In this example, each unit structure U2 includes a region that is surrounded by three loops in the wire member W2. Specifically, each unit structure U2 has a shape whose vertices are the bends b2 and the wire member intersections (the above-described intersections of the wire members W2). In this example, as illustrated in FIG. 2, the shape of each unit structure U2 further includes three regions each having a substantially diamond shape. Moreover, in this example, the unit structure U2 has a length along the axial direction Z (an axial direction length L2) that substantially matches a wave height of each wire member W2. The axial direction length L2 will be described later.

It is to be noted that such a unit structure U2 corresponds to a specific example of a "unit structure" in the invention.

Here, in the mesh structure 112, the above-described unit structure U2 and the gap region Ag are alternately arranged along the axial direction Z, as illustrated in FIG. 2. Specifically, in the mesh structure 112, the gap region Ag that forms a wavy shape (zigzag shape) extending along the circumferential direction R is provided between segments that each include a plurality of unit structures U2 arranged along the circumferential direction R. In other words, in the mesh structure 112, the gap region Ag is provided between the mesh patterns of the respective rows, and the wire members W2 in the mesh patterns of different rows do not intersect each other.

Moreover, as illustrated in FIG. 2, such a unit structure U2, and the unit structure U1 in the above-described mesh structure 111 are arranged so as to overlap each other (superimposed arrangement), allowing their positions to be shifted from each other along each of the axial direction Z and the circumferential direction R. Thus, in the example illustrated in FIG. 2, each unit structure U1 is divided into four or more (mainly four in this example) regions by the mesh structure 112 (two loops in the wire member W1).

Moreover, as illustrated in FIG. 2, all of the coupling parts C11 and C12 formed in the mesh structure 111 are surrounded by the unit structure U2 (the wire member W2). In other words, in this stent 11, the coupling parts C11 and C12 each include only a coupling part that is surrounded by the unit structure U2, and a coupling part that is not surrounded by the unit structure U2 is not provided.

Furthermore, as illustrated in FIG. 2, the length along the axial direction Z (the axial direction length L1) of the unit structure U1 and the length along the axial direction Z (the axial direction length L2) of the unit structure U2 have the following magnitude relationship. That is, the axial direction length L2 in the unit structure U2 is equal to or greater than the axial direction length L1 in the unit structure U1 (L2 ≥ L1), and particularly in this example, the axial direction length L2 is greater than the axial direction length L1 (L2 > L1).

It is to be noted that, in this example, the axial direction length L1 is about 8 mm to about 24 mm, for example, and the axial direction length L2 is about 8 mm to about 200 mm, for example. Moreover, it is desirable that a numerical range of a ratio of the axial direction length L2 with respect to the axial direction length L1 ((L2/L1) × 100) be about 100% to about 500%. It is to be noted that the axial direction length L2 may be the same as the length along the axial direction Z of the stent 11 (L2 = L1).

Here, in the stent 11 of the present embodiment, as illustrated in FIG. 2, the following is achieved in the both ends along the axial direction Z (the both end regions Ae). That is, in the both ends along the axial direction Z, the wire member W1 constituting the mesh structure 111 (the above-described tubular structure) is disposed, whereas the wire member W2 constituting the mesh structure 112 is not disposed. In other words, only the wire member W1 is disposed in the both ends along the axial direction Z of the stent 11.

Moreover, in the stent 11, as illustrated in FIG. 2, the following is achieved in the above-described gap region Ag. That is, in this gap region Ag, the wire member W1 constituting the mesh structure 111 (the tubular structure) is disposed, whereas the wire member W2 constituting the mesh structure 112 is not disposed. In other words, also in the gap region Ag of the stent 11, only the wire member W1 is disposed as in the both end regions Ae.

[Workings and Effects]

(Basic Operation)

During treatment of a tumor or the like near a digestive tract of a patient, the stent 11 is placed at a part to be treated (e.g., in the digestive tract such as a large intestine), making it possible to push open a lumen of the digestive tract that is narrowed by the tumor.

At this occasion, specifically, the stent 11 is first inserted into a predetermined delivery sheath in a state of being reduced in diameter, and the delivery sheath is inserted into the digestive tract to allow the stent 11 to be carried to the vicinity of an affected site. The stent 11 is then expanded by being deployed from within the delivery sheath. Thus, the stent 11 is placed in the affected site (the part to be treated).

(Workings and Effects of Stent 11)

Next, with reference to FIGs. 1 and 2, workings and effects of the stent 11 will be described in detail in comparison with a comparative example.

First, in the stent 11 of the present embodiment, the mesh structure 112 including the wire member W2 is formed in addition to the mesh structure 111 including the wire member W1, as illustrated in FIG. 2. Thus, in the stent 11, each unit structure U1 in the mesh structure 111 is divided into mainly four regions by the mesh structure 112 (the wire member W2). In other words, in this stent 11, the entire stent 11 has a smaller (finer) mesh because the mesh structure 112 is provided in addition to the mesh structure 111.

Moreover, in the stent 11, the wire members W1 are coupled to each other (the coupling parts C11 and C12 are formed) in the mesh structure 111, whereas the wire members W2 in the mesh structure 112 are not coupled to each other, as illustrated in FIG. 2. In other words, although the wire members W2 intersect each other, a coupling part like the coupling parts C11 and C12 between the wire members W1 is not formed. This avoids an increase in the number of coupling parts due to the addition of the mesh structure 112 in the stent 11. As a result, in the stent 11, the wire member W2 in the mesh structure 112 is able to move (be displaced). Thus, when the stent 11 is curved, force repelling the curvature is less likely to occur. In addition, the number of coupling parts arranged along the circumferential direction R is reduced.

Incidentally, it is generally demanded that a stent be favorable in terms of various characteristics such as those described below (e.g., expansion force, conformability, diameter reducibility, migration resistance, deployment resistance, a shortening rate, and X-ray visibility).

(1) Expansion force expanding a narrowed part of a digestive tract (a part to be treated) or a blood vessel
(2) A characteristic of following a curved shape of the digestive tract or the blood vessel (conformability)
(3) Diameter reducibility in inserting the stent into the above-described delivery sheath
(4) Resistance to unintentional movement after stent placement (migration resistance)
(5) Deployment resistance (a magnitude of force that is necessary to retract the delivery sheath)
(6) A shortening rate (representing a degree of change in the length in the axial direction Z when the stent is deployed (a normal state) from a state in which the stent is housed in the delivery sheath (a mounted state), and being calculated by the following expression (A))

$$\text{The shortening rate} = [\{(\text{the length in the axial direction } Z \text{ of the stent in the state of being mounted in the delivery sheath}) - (\text{the length in the axial direction } Z \text{ of the stent in the normal state})\}/ (\text{the length in the axial direction } Z \text{ of the stent in the state of being mounted in the delivery sheath})] \times 100... (A)$$

(7) X-ray visibility (visibility of the stent under fluoroscopy)
(8) Durability (resistance to fatigue fracture that is caused by peristaltic movement or the like after stent placement)
(9) Long-term stability (resistance to deformation that is caused in a case where the stent is mounted on the delivery sheath for a long period of time)

It is to be noted that, if the above-described coupling parts are provided, motions of the wire member are generally limited. Therefore, when the stent is curved, force repelling the curvature occurs in the coupling parts to impair the conformability. Furthermore, since each coupling part is formed by the bends in the wire member being engaged with each other, the wire member is concentrated in the vicinity of this coupling part. Therefore, if a large number of coupling parts are arranged along the circumferential direction of the stent, the diameter reducibility is also impaired.

Thus, it is generally demanded that a stent for a digestive tract have the above-described various characteristics set within appropriate ranges, but it is presumably very difficult to satisfy all the characteristics using a single (one type) wire member.

Accordingly, in the stent 11 of the present embodiment, the wire diameter r1 of the wire member W1 constituting the mesh structure 111 (the above-described tubular structure) is smaller than the wire diameter r2 of the wire member W2 constituting the mesh structure 112 (the wire diameter r1 < the wire diameter r2), as illustrated in FIG. 2. Thus, the stent 11 makes it easier to well-balance the various characteristics of the stent 11, which vary depending on the magnitudes of the wire diameters (the wire diameters r1 and r2). This is described in detail below.

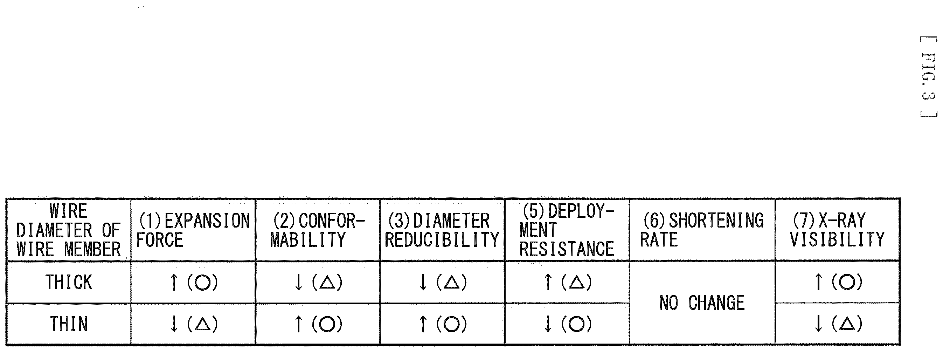

Here, FIG. 3 is a table illustrating an example of a correspondence between a wire diameter of a wire member and various characteristics in a typical stent. It is to be noted that the correspondence between the wire diameter of the wire member and the migration resistance has not been known so far. This will be described in detail later. It is to be noted that, in FIG. 3, "↑" means a change in which the characteristic value increases, and "↓" means a change in which the characteristic value decreases. Further, in FIG. 3, "○" enclosed in parentheses means that the change in the characteristic value is a change in a favorable (desirable) direction, and "△" enclosed in parentheses means that the change in the characteristic value is a change in a poor (undesirable) direction.

It is apparent from FIG. 3 that the expansion force and the X-ray visibility each increase to change in the favorable direction in response to the wire diameter of the wire member increasing (becoming thick). It is to be noted that, although not illustrated in FIG. 3, also the durability and the long-term stability each increase to change in the favorable direction in response to the increase in the wire diameter of the wire member. Moreover, it is apparent from FIG. 3 that the deployment resistance increases to change in the poor direction, and the conformability and the diameter reducibility each decrease to change in the poor direction in response to the increase in the wire diameter of the wire member.

On the other hand, it is apparent from FIG. 3 that, conversely, the expansion force and the X-ray visibility each decrease to change in the poor direction in response to the wire diameter of the wire member decreasing (becoming thin). It is to be noted that, although not illustrated in FIG. 3, also the durability and the long-term stability each decrease to change in the poor direction in response to the decrease in the wire diameter of the wire member. Moreover, it is apparent from FIG. 3 that the deployment resistance decreases to change in the favorable direction, and the conformability and the diameter reducibility each increase to change in the favorable direction in response to the decrease in the wire diameter of the wire member.

It is to be noted that, it is apparent from FIG. 3 that the shortening rate does not change even if the wire diameter of the wire member is changed. In other words, the shortening rate is a characteristic that is independent of the wire diameter of the wire member. Incidentally, it has been found that the shortening rate is greatly influenced by how the wire member in the stent is woven.

Here, as described above, the correspondence between the wire diameter of the wire member and the migration resistance has not been known so far. In contrast, the inventors of the present application have confirmed by study that, in stents that are formed by weaving in the same way, the above-described migration resistance increases in response to the wire diameter of the wire member increasing (becoming thick).

Moreover, the present inventors have confirmed that, in the stent 11 of the present embodiment that includes the two types of mesh structures 111 and 112 including the two types of wire members W1 and W2, the characteristics changes as follows in a case where either wire diameter is changed (see Reference Examples 1 to 3 below).

Description will be given with a specific example. In the stent 11 according to an example of the present embodiment (example: an example where r1 = 0.18 mm and r2 = 0.20 mm), the following was achieved as compared with the stent of Reference Example 1 where both the wire diameters r1 and r2 were 0.18 mm (r1 = r2 = 0.18 mm). That is, in the stent 11 of the example, the expansion force, the migration resistance, and the deployment resistance each increased, and the diameter reducibility and the conformability each decreased, as compared with the stent of Reference Example 1.

On the other hand, in the stent 11 according to the example (the example where r1 = 0.18 mm and r2 = 0.20 mm), the following was achieved as compared with the stent of Reference Example 2 where both the wire diameters r1 and r2 were 0.20 mm (r1 = r2 = 0.20 mm). That is, in the stent 11 of the example, the expansion force, the migration resistance, and the deployment resistance each decreased, and the diameter reducibility and the conformability each increased, as compared with the stent of Reference Example 2.

Moreover, in the stent 11 according to the example (the example where r1 = 0.18 mm and r2 = 0.20 mm), the following was achieved as compared with the stent of Reference Example 3 where both the wire diameters r1 and r2 were 0.19 mm (r1 = r2 = 0.19 mm). That is, in the stent 11 of the example, it was confirmed that the expansion force was substantially the same (does not change), whereas the deployment resistance decreased and the migration resistance increased, as compared with the stent of Reference Example 3.

Accordingly, the following is presumably achieved in the stent 11 of the present embodiment that includes the two types of mesh structures 111 and 112 including the two types of wire members W1 and W2 satisfying the magnitude relationship between the wire diameters r1 and r2 (r1 < r2). That is, for example, the stent 11 of the present embodiment makes it possible to increase the migration resistance, while maintaining low deployment resistance without changing the expansion force, as compared with the stent that includes the two types of mesh structures including the wire member of the intermediate value between the wire diameters r1 and r2 in the example (= (r1 + r2)/2) as in Reference Example 3.

From the above description, the following is achieved in the stent 11 of the present embodiment, because the wire diameter r1 of the wire member W1 constituting the mesh structure 111 (the above-described tubular structure, the main stent) extending over the entire length of the stent 11 is made relatively small (thin). That is, for example, the deployment resistance in the mesh structure 111 decreases to change in the favorable direction, as illustrated in FIG. 3. Moreover, when the delivery sheath is retracted to deploy the stent 11, high deployment resistance makes it necessary to apply force for retraction, which can prevent accurate operation and cause the stent to be displaced from the intended part during the placement. However, the deployment resistance of the stent 11 is kept low as described above, allowing the stent 11 to be placed in an accurate position.

Moreover, the following is achieved in the stent 11, because the wire diameter r2 of the wire member W2 constituting the additionally provided mesh structure 112 (sub-stent) is made relatively large (thick). That is, for example, the expansion force, the X-ray visibility, the durability, and the long-term stability each increase to change in the favorable direction, as illustrated in FIG. 3, etc. Moreover, as described above, the migration resistance increases to change in the favorable direction. Furthermore, a stent that is used for a digestive tract generally undergoes peristaltic movement of the digestive tract and passage of feces through an organ, which can cause migration over time after the stent placement. This can prevent sufficient expansion of the narrowed part and cause re-narrowing. In contrast, in the stent 11 of the present embodiment, since the migration resistance is high as described above, it is possible to keep sufficient expansion of the narrowed part even after elapse of a period of time after the placement of the stent 11.

As described above, the stent 11 of the present embodiment includes the two types of mesh structures 111 and 112 including the two types of wire members W1 and W2 satisfying such a magnitude relationship between the wire diameters r1 and r2 (r1 < r2). This makes it easier to well-balance the various characteristics of the stent 11, which vary depending on the magnitudes of the wire diameters (the wire diameters r1 and r2).

Specifically, the mesh structure 111 (main stent) including the wire member W1 having the thin diameter (the wire diameter r1) is provided over the entire length of the stent 11 to maintain low deployment resistance, which is important during the placement of the stent 11. Furthermore, the mesh structure 112 (sub-stent) including the wire member W2 having the thick diameter (the wire diameter r2) is provided partially and additionally to improve the migration resistance, the expansion force, and the like. As a result, the present embodiment enables the characteristics of the stent 11 to be improved. This makes it possible to keep sufficient expansion of the narrowed part of the digestive tract etc., while maintaining the stent 11 at the intended treatment position.

Moreover, in the both ends along the axial direction Z (the both end regions Ae) in the stent 11 of the present embodiment, the wire member W1 constituting the mesh structure 111 is disposed, whereas the wire member W2 constituting the mesh structure 112 is not disposed, as illustrated in FIG. 2. In other words, only the wire member W1 is disposed in the both ends along the axial direction Z of the stent 11. Thus, the expansion force of the stent 11 is weakened in the both ends, making the digestive tract less likely to be damaged in these both ends. This improves the reliability of the stent 11.

Furthermore, in the above-described gap region Ag in the stent 11 of the present embodiment, the wire member W1 constituting the mesh structure 111 is disposed, whereas the wire member W2 constituting the mesh structure 112 is not disposed, as illustrated in FIG. 2. In other words, also in the gap region Ag of the stent 11, only the wire member W1 is disposed as in the both ends (the both end regions Ae). This allows moderate conformability to the digestive tract (digestive tract conformability) to be maintained while the expansion force of the entire stent 11 is kept. This further improves the characteristics of the stent 11.

Incidentally, it is desirable that such a gap region Ag have a width (a length of the gap region Ag along the axial direction Z) in a moderate range, i.e., the desirable length of the gap region Ag along the axial direction Z is neither too long nor too short. Specifically, for example, to prevent the mesh structure 112 from making the entire stent 11 difficult to deform, the gap region Ag may be formed to the extent that the above-described segments formed in the circumferential direction R by the plurality of unit structures U2 do not overlap (intersect) each other along the axial direction Z.

As described above, in the present embodiment, the following is achieved because, in the stent 11, the wire diameter r1 of the wire member W1 constituting the mesh structure 111 is made smaller than the wire diameter r2 of the wire member W2 constituting the mesh structure 112. That is, it is possible to well-balance the various characteristics of the stent 11, which vary depending on the magnitudes of the wire diameters, as described above. Therefore, the present embodiment makes it possible to improve the characteristics of the stent 11.

It is to be noted that, in the present embodiment, the axial direction length L2 in the unit structure U2 is equal to or greater than the axial direction length L1 in the unit structure U1 ($L2 \geq L1$) as illustrated in FIG. 2, but this is non-limiting. For example, conversely, the axial direction length L2 in the unit structure U2 may be less than the axial direction length L1 in the unit structure U1 ($L2 < L1$). In this case, it is desirable that the axial direction length L2 be, for example, about 2 mm to about 23 mm, and that the numerical range of the ratio of the axial direction length L2 to the axial direction length L1 (($L2/L1$) $\times$ 100) be about 25% to about 95%.

Moreover, in the present embodiment, all of the plurality of the coupling parts C11 and C12 formed in the mesh structure 111 are surrounded by the unit structure U2 (the wire member W2) as illustrated in FIG. 2, but this is non-limiting. For example, at least some of the plurality of coupling parts C11 and C12 formed in the mesh structure 111 may not be surrounded by the unit structure U2 (the wire member W2). In other words, the coupling parts C11 and C12 may include two types of coupling parts, i.e., a coupling part that is surrounded by the unit structure U2 and a coupling part that is not surrounded by the unit structure U2.

Furthermore, the present embodiment has described an example case where the coupling part C12 is formed closer to the outer circumference than the intersection in the mesh pattern is, but this is non-limiting. In some cases, conversely, the coupling part C12 may be formed closer to an inner circumference than the intersection in the mesh pattern is.

In addition, in the present embodiment, the coupling parts C12 are formed from starting points that are the both ends (the both end regions Ae) in the both ends along the axial direction Z of the mesh structure 111, but this example is non-limiting. For example, the coupling parts C12 may be formed from a starting point that is only one end (one end region Ae) of the both ends.

Moreover, the present embodiment has described a case where the mesh pattern of the predetermined odd-numbered row constituting the coupling part C12 is the mesh pattern of the third or fifth row (the third row in the example of FIG. 2). That is, the coupling part C12 is formed by the bend b1 in the mesh pattern of the first row and the bend b1 in the mesh pattern of the third or fifth row being coupled to each other. However, this example is non-limiting. For example, the mesh pattern of the predetermined odd-numbered row may be the seventh or subsequent odd-numbered row (the coupling part C12 may be formed by the bend b1 in the mesh pattern of the first row and the bend b1 in the mesh pattern of the seventh or subsequent odd-numbered row being coupled to each other).

<2. Modification Example>

Next, a modification example of the above embodiment will be described. Specifically, in the following modification example, description will be given on another configuration example of the stent according to the invention (a configuration example of a stent having another mesh structure that is different from that in the embodiment). It is to be noted that, in this modification example, the same components as those in the embodiment are denoted by the same reference numerals, and the description thereof is omitted as appropriate.

[Outline Configuration]

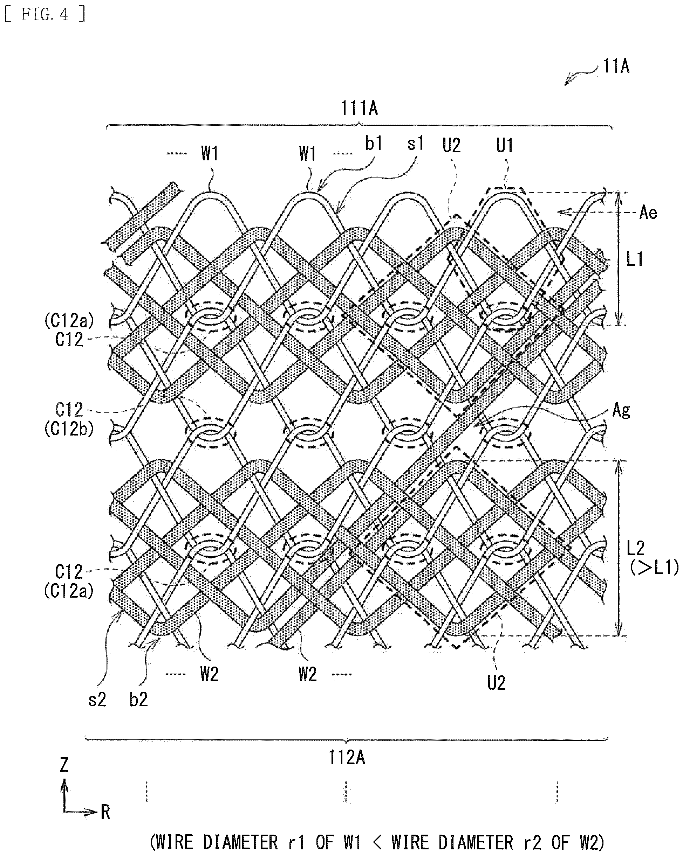

FIG. 4 is a schematic development view of a configuration example of a main part (a region near both ends) of a stent (a stent 11A) according to the modification example. The stent 11A corresponds to the stent 11 of the embodiment illustrated in FIG. 2 in which the above-described mesh structures 111 and 112 are replaced with mesh structures 111A and 112A to be described below.

It is to be noted that, also in FIG. 4, for convenience, the mesh structure 111A including the wire member W1 is not hatched while the mesh structure 112A including the wire member W2 is hatched, as in FIG. 2.

Moreover, these mesh structures 111A and 112A respectively correspond to specific examples of a "first mesh structure" (main stent) and a "second mesh structure" (sub-stent) in the invention.

Here, as illustrated in FIG. 4, firstly, the stent 11A of the present modification example includes the two types of mesh structures 111A and 112A, like the stent 11 of the embodiment (see FIG. 2). That is, the stent 11A also includes the mesh structure 111A including the wire member W1 and the mesh structure 112A including the wire member W2. Of these, the mesh structure 111A constitutes the above-described tubular structure and extends over the entire length in the axial direction Z of the stent 11A. In other words, this mesh structure 111A has no gap region along the axial direction Z, unlike the mesh structure 112A. In contrast, the mesh structure 112A is disposed in at least a portion (a portion in the example of FIG. 4) of the stent 11A, and has a gap region Ag along the axial direction Z as illustrated in FIG. 4, like the mesh structure 112.

Moreover, also in the stent 11A of the present modification example, as illustrated in FIG. 4, the wire members W1 and W2 respectively constituting such two types of mesh structures 111A and 112A have the wire diameters r1 and r2 that are different from each other (the wire diameter r1 of the wire member W1 ≠ the wire diameter r2 of the wire member W2). In other words, the stent 11A includes the two types of wire members W1 and W2 with the different wire diameters r1 and r2, like the stent 11. Moreover, particularly in the stent 11A of the present modification example, as illustrated in FIG. 4, the wire diameter r1 of the wire member W1 constituting the mesh structure 111A (the tubular structure) is smaller (thinner) than the wire diameter r2 of the wire member W2 constituting the mesh structure 112A (the wire diameter r1 < the wire diameter r2). To put it another way, the wire diameter r2 of the wire member W2 is larger (thicker) than the wire diameter r1 of the wire member W1.

[Detailed Configurations of Mesh Structures 111A and 112A]

Hereinafter, with reference to FIG. 4, detailed configurations of the mesh structures 111A and 112A in the present modification example will be described.

Like the mesh structure 111, the mesh structure 111A is formed by the wire member W1 intersecting at its straight parts s1. The wire member W1 includes the straight parts s1 and the bends b1 and forms a wavy shape. Therefore, in the mesh structure 111A, intersections (wire member intersections) are formed, each of which is a portion where the straight parts s1 of the wire member W1 intersect each other. Moreover, like the mesh structure 112, the mesh structure 112A is formed by the wire member W2 intersecting at its straight parts s2. The wire member W2 includes the straight parts s2 and the bends b2 and forms a wavy shape. Therefore, also in the mesh structure 112A, intersections (wire member intersections) are formed, each of which is a portion where the straight parts s2 of the wire member W2 intersect each other. Moreover, the mesh structure 112A is woven into the mesh structure 111A, by the straight part s1 of the wire member W2 and the straight part s2 of the wire member W1 intersecting each other.

Moreover, like the mesh structure 111, the mesh structure 111A includes the plurality of unit structures U1 that is two-dimensionally arranged side by side along each of the axial direction Z and the circumferential direction R, as illustrated in FIG. 4. In addition, like the mesh structure 112, the mesh structure 112A includes the plurality of unit structures U2 that is two-dimensionally arranged side by side along each of the axial direction Z and the circumferential direction R.

In this example, each unit structure U1 includes a region that is surrounded by two wire members W1. Specifically, each unit structure U1 has a substantially diamond shape whose long axis direction is the axial direction Z and short axis direction is the circumferential direction R, and whose vertices are two bends b1 and two wire member intersections (the above-described intersections of the wire members W1). Therefore, in this example, the unit structure U1 has a length along the axial direction Z (an axial direction length L1) that matches a wave height of each wire member W1 (a length in the axial direction Z of the above-described wavy shape). The axial direction length L1 will be described later.

On the other hand, in this example, each unit structure U2 includes a region that is surrounded by two wire members W2. Specifically, each unit structure U2 has a substantially diamond shape whose long axis direction is the axial direction Z and short axis direction is the circumferential direction R, and whose vertices are two bends b2 and two wire member intersections (the above-described intersections of the members W2). Therefore, in this example, the unit structure U2 has a length along the axial direction Z (an axial direction length L2) that matches a wave height of each wire member W2. The axial direction length L2 will be described later. Moreover, in this example, the substantially diamond shape in the unit structure U2 further includes four regions each having a substantially diamond shape, as illustrated in FIG. 4.

Here, as illustrated in FIG. 4, in the mesh structure 112A, the above-described unit structure U2 and the gap region Ag are alternately arranged along the axial direction Z, as in the mesh structure 112. Specifically, in the mesh structure 112A, the gap region Ag forming a wavy shape (zigzag shape) extending along the circumferential direction R is provided between segments each including a plurality of unit structures U2 arranged along the circumferential direction R. In other words, in the mesh structure 112A, the gap region Ag is provided between the mesh patterns of the respective rows, and the wire members W2 in the mesh patterns of different rows do not intersect each other.

Moreover, as illustrated in FIG. 4, these unit structures U1 and U2 are arranged so as to overlap each other (superimposed arrangement), allowing their positions to be shifted from each other along each of the axial direction Z and the circumferential direction R. Thus, in the example illustrated in FIG. 4, each unit structure U1 is divided into four or more (mainly four in this example) regions by the mesh structure 112A (two wire members W2).

Here, in the stent 11A of the present modification example, the coupling parts C12 (hooking parts) including the wire members W1 coupled to (engaged with) each other at the bends b1 are formed in the mesh structure 111A, as illustrated in FIG. 4. That is, the mesh structure 111A includes mesh patterns that are coupled together along the axial direction Z. Each of the mesh patterns is formed by causing the wire member W1 that includes the straight parts s1 and the bends b1 and forms a wavy shape to progress along the circumferential direction R. In contrast, the wire members W2 intersect each other, but are not coupled to each other at the bends b2 (a coupling part like the coupling parts C12 is not formed).

Moreover, at least some of the plurality of coupling parts C12 formed in the mesh structure 111A are not surrounded by the unit structure U2 (the wire member W2). Specifically, in this example, the coupling parts C12 include two types of coupling parts, i.e., a coupling part C12a that is surrounded by the unit structure U2 and a coupling part C12b that is not surrounded by the unit structure U2, as illustrated in FIG. 4.

Furthermore, in this example, as illustrated in FIG. 4, the length along the axial direction Z (the axial direction length L1) of the unit structure U1 and the length along the axial direction Z (the axial direction length L2) of the unit structure U2 have the following magnitude relationship. That is, the axial direction length L2 in the unit structure U2 is equal to or greater than the axial direction length L1 in the unit structure U1 (L2 $\geq$ L1), and particularly in the present modification example, the axial direction length L2 is greater than the axial direction length L1 (L2 > L1).

It is to be noted that, in this example, the axial direction length L1 is about 8 mm to about 24 mm, for example, and the axial direction length L2 is about 8 mm to about 200 mm, for example. Moreover, it is desirable that a numerical range of a ratio of the axial direction length L2 with respect to the axial direction length L1 ((L2/L1) $\times$ 100) be about 100% to about 500%. It is to be noted that the axial direction length L2 may be the same as the length along the axial direction Z of the stent 11A.

Here, in the stent 11A of the present modification example, the following is achieved in the both ends along the axial direction Z (the both end regions Ae), as illustrated in FIG. 4. That is, in the both ends along the axial direction Z, the wire member W1 constituting the mesh structure 111A (the above-described tubular structure) is disposed, whereas the wire member W2 constituting the mesh structure 112A is not disposed, as in the stent 11. In other words, only the wire member W1 is disposed in the both ends along the axial direction Z of the stent 11A.

Moreover, in the stent 11A, the following is achieved in the above-described gap region Ag, as illustrated in FIG. 4. That is, in this gap region Ag, the wire member W1 constituting the mesh structure 111A (the tubular structure) is disposed, whereas the wire member W2 constituting the mesh structure 112A is not disposed, as in the stent 11. In other words, also in the gap region Ag of the stent 11A, only the wire member W1 is disposed as in the both end regions Ae.

[Workings and Effects]

In the stent 11A of the present modification example having such a configuration (the mesh structures 111A and 112A), it is possible to obtain similar effects by workings similar to those of the stent 11 of the embodiment.

That is, the following is achieved also in the stent 11A because, as illustrated in FIG. 4, the wire diameter r1 of the wire member W1 constituting the mesh structure 111A is made smaller than the wire diameter r2 of the wire member W2 constituting the mesh structure 112A, as in the stent 11. In other words, it is possible to well-balance the various characteristics of the stent 11A, which vary depending on the magnitudes of the wire diameters, as in the case of the above-described stent 11. Therefore, the present modification example also makes it possible to improve the characteristics of the stent 11A.

Moreover, the following is achieved also in the stent 11A because, as illustrated in FIG. 4, only the wire member W1 constituting the mesh structure 111A is disposed in the both ends along the axial direction Z (the both end regions Ae), as in the stent 11. That is, the expansion force of the stent 11A is weakened in the both ends, making the digestive tract less likely to be damaged in these both ends. Therefore, also in the present modification example, it is possible to improve the reliability of the stent 11A.

Furthermore, the following is achieved also in the stent 11A because, as illustrated in FIG. 4, only the wire member W1 constituting the mesh structure 111A is disposed in the gap region Ag, as in the stent 11. That is, moderate conformability (digestive tract conformability) is maintained while the expansion force of the entire stent 11A is kept.

Therefore, also in the present modification example, it is possible to further improve the characteristics of the stent 11A.

<3. Application Example>

Next, description will be given on an example of applying the above-described stents (the stents 11 and 11A) according to the embodiment and the modification example to a medical device.

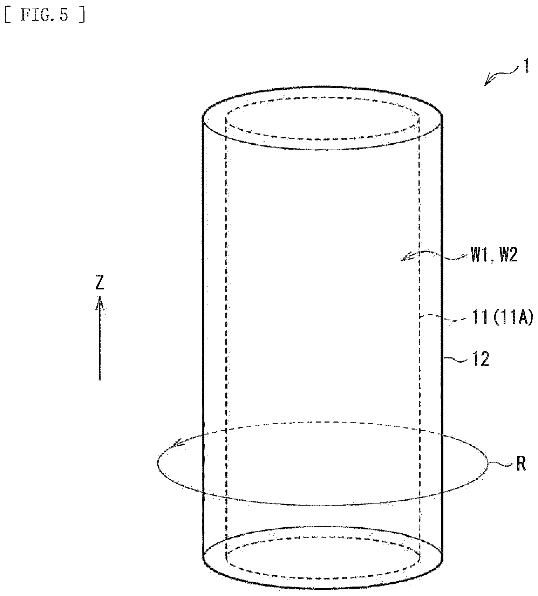

FIG. 5 is a schematic perspective view of an outline configuration example of a medical device (a medical device 1) according to the present application example. The medical device 1 includes the stent 11 or 11A and a tubular member 12 to be described below. This medical device 1 is a device to be applied, for example, to a tubular organ in a body, such as a digestive tract or a blood vessel. Specifically, the medical device 1 is, for example, a medical device such as a covered stent or a stent graft that is to be adapted to the blood vessel for treatment of aortic dissection, etc.

(Tubular Member 12)

The tubular member 12 has a tubular (cylindrical) shape as illustrated in FIG. 5. The tubular member 12 is disposed so as to cover (coat) part or all of the stent 11 (11A). Specifically, the tubular member 12 is disposed so as to cover an outer circumference side of the stent 11 (11A) in this example.

Moreover, the tubular member 12 is coupled to the stent 11 (11A) by a method such as sewing, attaching, or welding, so as to coat the stent 11 (11A). It is to be noted that a coupling part at which the tubular member 12 and the stent 11 (11A) are coupled to each other is provided as appropriate at a location such as the end region Ae or the intermediate region of the stent 11 (11A).

Here, in this example, the stent 11 (11A) is disposed in the entire region (the end region Ae and the intermediate region) along the axial direction Z of the tubular member 12. However, this is non-limiting, and the stent 11 (11A) may be disposed only in a partial region along the axial direction Z of the tubular member 12. In other words, the medical device 1 may include, along its axial direction Z, a region (stent disposed region) in which the stent 11 (11A) is disposed and a region (stent non-disposed region) in which the stent 11 (11A) is not disposed.

Examples of such a tubular member 12 include: a resin that is formed into a tubular shape by a molding method such as extrusion molding or blow molding; a tubular-shaped knitted or woven material including a fiber of resin or a super-fine metal wire; a tubular-shaped non-woven fabric including a resin or super-fine metal; a tubular-shaped resin sheet or a tubular-shaped porous sheet; a structure derived from a resin that has been dissolved in a solvent and is formed into a thin tubular shape; etc.

Here, as the above-described knitted or woven material, a publicly-known material knitted or woven in plane weave, twill weave, etc. may be used. Alternatively, a knitted or woven material with a crimp such as that subjected to a crimping process may also be used.

Moreover, as the above-described resin, for example, a resin having durability and causing a small tissue reaction may be used. Examples of such a resin include: polyolefin such as polyethylene, polypropylene, or an ethylene-$\alpha$-olefin copolymer; polyester such as polyamide, polyurethane, polyethylene terephthalate, polybutylene terephthalate, polycyclohexane terephthalate, or polyethylene-2, 6-naphthalate; vinyl resin such as polyvinyl chloride, vinyl acetate, or an ethylene-vinyl acetate copolymer; fluororesin such as polyethylene fluoride or polypropylene fluoride, etc. Examples of such a resin also include polyamide, polyamide elastomer, polyurethane, silicone resin, natural rubber, etc. It is to be noted that, among the above-described materials, the polyester such as polyethylene terephthalate, the fluororesin such as polyethylene fluoride or polypropylene fluoride, or silicone resin that are chemically stable, have great durability, and cause a small tissue reaction may be preferably used in particular.

Also in the medical device 1 of the present application example, it is basically possible to obtain similar effects by workings similar to those of the embodiment and the modification example.

Moreover, it is possible to obtain the following effects, for example, particularly in a case of a stent graft to be applied to a blood vessel for treatment of an aortic aneurysm or aortic dissection, etc. That is, in a case of such a stent graft to be applied to the blood vessel, high deployment resistance can prevent accurate operation during placement, causing the stent graft to deviate from the intended part, or migration can cause the stent graft to move from a part where it has been placed. If this happens, blood can flow into the aneurysm to cause a rupture. Moreover, blood can flow into a false lumen, causing the adventitia of the blood vessel to tear, resulting in bleeding. These suggest that it is clinically effective to achieve ease of retraction from the delivery sheath and high migration resistance, as in the medical device 1 of the present application example.

<4. Other Modification Examples>

The invention has been described above with reference to the embodiment, the modification example, and the application example; however, the invention is not limited to the embodiment, etc. described above, and is modifiable in a variety of ways.

For example, shapes, locations, sizes, numbers, materials, etc., of the respective members described in the foregoing embodiment, etc. are non-limiting, and may respectively be any other shape, location, size, number, material, etc. Specifically, for example, the tubular member may cover the inner circumference side of the stent or may cover both the inner circumference side and the outer circumference side of the stent.

Moreover, the arranged shape (the weaving pattern) of the wire members of the stent is not limited to those referred to in the foregoing embodiment, and may be any other arranged shape.

Furthermore, although the foregoing application example has been described with reference to an example case where only one stent is disposed in the medical device, this is non-limiting. Two or more stents may be individually disposed in the medical device (for example, the two or more stents may be disposed along the axial direction Z while being separated from each other).

In addition, the foregoing embodiment, etc. have described a case where the stent includes two types of wire members (wires) having wire diameters different from each other, but this case is non-limiting. That is, for example, the stent may include three or more types of wire members (wires) having wire diameters different from each other. In that case, specifically, the "second wire member" constituting the "second mesh structure" (sub-stent) in the invention may include a plurality of types (two or more types) of wire members (wires) having wire diameters different from each other, for example.

Moreover, although the foregoing embodiment, etc. have been mainly described with reference to examples of the stent and the medical device that are applied to the treatment of the digestive tract such as the large intestine, this is non-limiting. That is, each of the stent and the medical device of the invention is also applicable to treatment of another digestive tract other than the large intestine, or of a tubular organ in the body other than the digestive tract, for example, a blood vessel such as an aorta.

**Claims**

1. A stent to be applied to a tubular organ in a body, the stent comprising:

   a first mesh structure that includes one or more first wire members and has a tubular structure extending over an entire length in an axial direction of the stent; and
   a second mesh structure that is disposed in at least a portion of the stent and includes one or more second wire members intersecting the first wire members of the first mesh structure,
   wherein the first wire members are coupled to each other to form a coupling part in the first mesh structure, and the second wire members are not coupled to each other in the second mesh structure, and
   the first wire member has a wire diameter that is smaller than a wire diameter of the second wire member.

2. The stent according to claim 1, wherein, in both ends along the axial direction of the stent, the first wire member constituting the first mesh structure is disposed, and the second wire member constituting the second mesh structure is not disposed.

3. The stent according to claim 1 or 2, wherein
   the second mesh structure includes a unit structure and a gap region that are provided alternately along the axial direction of the stent, and,
   in the gap region, the first wire member constituting the first mesh structure is disposed, and the second wire member constituting the second mesh structure is not disposed.

4. A medical device comprising:

   a tubular member; and
   at least one stent disposed in at least a portion of the tubular member,
   the stent including

   a first mesh structure that includes one or more first wire members and has a tubular structure extending over an entire length in an axial direction of the stent, and
   a second mesh structure that is disposed in at least a portion of the stent and includes one or more second wire members intersecting the first wire members of the first mesh structure,

   wherein the first wire members are coupled to each other to form a coupling part in the first mesh structure, and the second wire members are not coupled to each other in the second mesh structure, and
the first wire member has a wire diameter that is smaller than a wire diameter of the second wire member.

[ FIG. 1 ]

[ FIG. 2 ]

(WIRE DIAMETER r1 OF W1 < WIRE DIAMETER r2 OF W2)

[FIG. 3]

| WIRE DIAMETER OF WIRE MEMBER | (1) EXPANSION FORCE | (2) CONFOR-MABILITY | (3) DIAMETER REDUCIBILITY | (5) DEPLOY-MENT RESISTANCE | (6) SHORTENING RATE | (7) X-RAY VISIBILITY |
|---|---|---|---|---|---|---|
| THICK | ↑ (○) | ↓ (△) | ↓ (△) | ↑ (△) | NO CHANGE | ↑ (○) |
| THIN | ↓ (△) | ↑ (○) | ↑ (○) | ↓ (○) | | ↓ (△) |

[ FIG. 4 ]

(WIRE DIAMETER r1 OF W1 < WIRE DIAMETER r2 OF W2)

[ FIG. 5 ]

<div align="center">

**INTERNATIONAL SEARCH REPORT**

</div>

| | International application No. |
|---|---|
| | PCT/JP2018/016040 |

**A. CLASSIFICATION OF SUBJECT MATTER**
Int. Cl. A61F2/90(2013.01)i

According to International Patent Classification (IPC) or to both national classification and IPC

**B. FIELDS SEARCHED**

Minimum documentation searched (classification system followed by classification symbols)
Int. Cl. A61F2/90

Documentation searched other than minimum documentation to the extent that such documents are included in the fields searched
Published examined utility model applications of Japan     1922-1996
Published unexamined utility model applications of Japan   1971-2018
Registered utility model specifications of Japan           1996-2018
Published registered utility model applications of Japan   1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2017-47003 A (JAPAN LIFELINE CO., LTD.) 09 March 2017, abstract, paragraphs [0020]-[0032], [0072], fig. 1, 2 & WO 2017/038145 A1 & KR 10-2017-0139086 A | 1-4 |
| Y | JP 2001-212246 A (SHIMADZU CORP.) 07 August 2001, abstract, paragraphs [0011]-[0015], fig. 1, 2 (Family: none) | 1-4 |
| Y | JP 2017-29387 A (JMS CO., LTD., UNIVERSITY KYOTO) 09 February 2017, abstract, paragraphs [0005], [0015]-[0023], fig. 1 (Family: none) | 1-4 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01.06.2018 | 12.06.2018 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**EP 3 643 274 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009501049 W